# EUROPÄISCHE PATENTSCHRIFT

(11) **EP 1 392 243 B1**
(45) Veröffentlichungstag und Bekanntmachung des Hinweises auf die Patenterteilung: **27.02.2019**
(21) Anmeldenummer: 02729814.0
(22) Anmeldetag: 25.03.2002
(51) Int. Cl.: A61K 9/127, A61K 47/18, A61K 47/24, A61K 31/205, A61K 47/44, A61K 31/195, A61K 31/165, A61K 38/28, A61P 29/02, A61P 23/00, A61P 3/10

(54) **PHARMAZEUTISCHE ZUSAMMENSETZUNG**
PHARMACEUTICAL COMPOSITION
COMPOSITION PHARMACEUTIQUE

(30) Priorität: 10.05.2001 DE 10122772; 27.07.2001 DE 10136776
(43) Veröffentlichungstag der Anmeldung: 03.03.2004
(73) Patentinhaber: Kuhs GmbH, 80339 München (DE)
(72) Erfinder: ALBRECHT, Martin, 51519 Odenthal (DE); MINTEL, Hermann, 40764 Langenfeld (DE)
(74) Vertreter: Vossius & Partner Patentanwälte Rechtsanwälte mbB
(86) Internationale Anmeldenummer: PCT/DE2002/001070
(87) Internationale Veröffentlichungsnummer: WO 2002/089770

(56) Entgegenhaltungen:
- WO-A-00/18371
- WO-A-01/62222
- WO-A-91/04013
- WO-A-98/44909
- DE-A- 19 839 441
- FR-A- 2 627 385
- US-A- 6 015 574
- SANTUS G C ET AL: "TRANSDERMAL ENHANCER PATENT LITERATURE" JOURNAL OF CONTROLLED RELEASE, ELSEVIER SCIENCE PUBLISHERS B.V. AMSTERDAM, NL, Bd. 25, Nr. 1 / 2, 27. Mai 1993 (1993-05-27), Seiten 1-20, XP000361364 ISSN: 0168-3659
- HERZOG R: "TRANSFERSOMEN - EIN NEUES TRANSPORTVEHIKEL FUER ARZNEISTOFFE?" DEUTSCHE APOTHEKER ZEITUNG, DEUTSCHER APOTHEKER ZEITUNG, STUTTGART, DE, Bd. 141, Nr. 34, 23. August 2001 (2001-08-23), Seiten 42-47, XP001119922 ISSN: 0011-9857
- CEVC G ET AL: "New, highly efficient formulation of diclofenac for the topical, transdermal administration in ultradeformable drug carriers, Transfersomes." BIOCHIMICA ET BIOPHYSICA ACTA. NETHERLANDS 1 OCT 2001, Bd. 1514, Nr. 2, 1. Oktober 2001 (2001-10-01), Seiten 191-205, XP004319625 ISSN: 0006-3002

## Beschreibung

Die vorliegende Erfindung betrifft eine pharmazeutische Zusammensetzung gemäß Patentanspruch 1 sowie eine derartige pharmazeutische Zusammensetzung enthaltende transdermal zu verabreichende Zubereitung.

In den letzten 20 Jahren wurden insbesondere pharmazeutische Zusammensetzungen, die topisch anzuwenden sind, entwickelt, wobei diese bekannten pharmazeutischen Zusammensetzungen, die durch eine Vielzahl von Schutzrechten bzw. Schutzrechtsanmeldungen beschrieben sind, neben Wasser und einem topisch applizierbaren Wirkstoff desweiteren mindestens eine Trägersubstanz für den Wirkstoff aufweisen. Hierbei dient diese Trägersubstanz dem Transport des Wirkstoffes durch die Hautstrukturen, so daß eine so angewandte pharmazeutische Zusammensetzung unter Vermeidung des Magen-Darm-Traktes und unter Vermeidung einer entsprechenden Injektion den Wirkstoff dorthin transportiert, wo er pharmazeutisch seine Wirkung lokal und/oder systemisch entfalten soll. Um diesen Transport des Wirkstoffes durch die Hautstrukturen zu beschleunigen und hierbei eine unerwünschte Zersetzung des Wirkstoffes zu verhindern, wird bei den bekannten pharmazeutischen Zusammensetzungen der Wirkstoff in der als Vesikel vorliegenden Trägersubstanz eingekapselt oder hieran angelagert, wobei derartige kugelförmige oder kugelige Vesikel als Liposome oder auch als Transfersome bezeichnet werden.

WO 01/62222 A2 beschreibt eine kosmetische Zusammensetzung, insbesondere zur Verwendung auf alternder und/oder gestresster Haut, wobei die Zusammensetzung zusätzlich zu Wasser mindestens eine Substanz enthält, die mit Wasser lamellare Strukturen bildet. Die Zusammensetzung umfasst weiterhin (a) mindestens eine Verbindung, die eine funktionelle Gruppe der allgemeinen Formel (I) -CH₂-N⁺-(CH₃)₃ enthält; (b) und/oder mindestens einen Metaboliten der Verbindung; (c) und/oder S-Adenosylmethionin.

FR 2 627 385 offenbart dermatologische und kosmetische Basen welche hydratisierte lipidische lamellare Phasen oder Liposomen-Vesikel enthalten.

EP 0 620 000 A2 beschreibt die Verwendung von mindestens einem N-Acyl-Alkanolamin-Derivat der allgemeinen Formel I (CH(R₂)(R₃)-(CH₂)ₙ-NH-R₁) als Wirkstoff in kosmetischen und/oder pharmazeutischen Zubereitungen zur Verhinderung von durch Lichtstrahlung verursachten Hautschäden und/oder zur Behandlung von durch Licht und/oder Wärme oder Insektenstichen hervorgerufenen Hautschäden.

Darüber hinaus können in einer derartigen bekannten pharmazeutischen Zusammensetzung noch weitere Inhaltsstoffe, wie z.B. Verdickungsmittel, Gelbildner, Farbstoffe, Stabilisatoren, Alterungsinhibitoren und/oder Duftstoffe sowie pH-Wert-Regulatoren,
enthalten sein.

Der vorliegenden Erfindung liegt die Aufgabe zugrunde, eine pharmazeutische Zusammensetzung zur Verfügung zu stellen, die eine besonders hohe pharmazeutische Wirksamkeit besitzt.

Diese Aufgabe wird erfindungsgemäß durch eine pharmazeutische Zusammensetzung mit den kennzeichnenden Merkmalen des Patentanspruchs 1 gelöst.

Die hierin offenbarte Zusammensetzung, die Wasser, mindestens einen topisch applizierbaren Wirkstoff und mindestens eine Trägersubstanz für den Wirkstoff aufweist, sieht vor, daß die Trägersubstanz eine mit Wasser eine lamellare Doppelmembranstruktur ausbildende Substanz ist und diese lamellare Doppelmembranstruktur in der offenbarten pharmazeutischen Zusammensetzung vorliegt. Desweiteren ist in der offenbarten Zusammensetzung mindestens eine Verbindung, die wenigstens eine funktionelle Gruppe der allgemeinen Formel I besitzt

- CH₂ - N^{⊕} - (CH₃)₃ (Formel I),

und/oder mindestens ein Metabolit dieser Verbindung und/oder S-Adenosylmethionin enthalten, wobei in der offenbarten Zusammensetzung mindestens ein N-Acyl-Ethanolamin vorgesehen ist.

Mit anderen Worten umfaßt somit die offenbarte pharmazeutische Zusammensetzung im einfachsten Fall neben Wasser, mindestens eine, mit Wasser eine lamellare Doppelmembranstruktur ausbildende Trägersubstanz und noch die zuvor genannte Verbindung, die die vorstehend aufgeführte funktionelle Gruppe der allgemeinen Formel I besitzt, und/oder einen Metaboliten dieser Verbindung und/oder S-Adenosylmethionin, wobei sichergestellt sein muß, daß in der offenbarten Zusammensetzung mindestens ein N-Acyl-Ethanolamin vorgesehen ist.

Die in der erfindungsgemäßen pharmazeutischen Zusammensetzung enthaltene Trägersubstanz bildet in der erfindungsgemäßen Zusammensetzung mit Wasser derartige lamellare Doppelmembranstruktur aus, die einen Schichtaufbau besitzen. Jeweils eine obere Schicht der Trägersubstanz ist zu einer unteren Schicht der Trägersubstanz ausgerichtet. Hierbei erfolgt diese Ausrichtung der einzelnen Trägersubstanz-Schichten derart, daß die hydrophilen Reste der Trägersubstanz jeweils nach außen und somit zu der diese Trägersubstanz-Schichten umgebenden wäßrigen Phase weisen, während die lipophilen Reste der Trägersubstanz zueinander nach innen ausgerichtet sind, was allgemein als Doppelmembran bezeichnet wird. Orientieren sich abhängig von der Konzentration der Trägersubstanz in der erfindungsgemäßen Zusammensetzung dann mindestens zwei Doppelmembranschichten sandwichartig übereinander, so entstehen geschichtete Doppelmembranen, wobei diese geschichteten Doppelmembranen beispielsweise zwei bis zehn parallel zueinander ausgerichtete, überwiegend planar verlaufende und jeweils durch mindestens eine Wasserschicht getrennte Doppelmembrane enthalten. Sowohl die zuvor beschriebene Doppelmembran als auch die geschichteten Doppelmembranen können als lamellare Struktur in der erfindungsgemäßen Zusammensetzung vorliegen und werden in der vorliegenden Anmeldung zusammengefaßt als lamellare Doppelmembranstruktur bezeichnet. Klarstellend ist anzumerken, daß in der erfindungsgemäßen Zusammensetzung neben dieser lamellaren Doppelmembranstruktur auch andere Strukturen, so beispielsweise die zuvor beschriebenen vesikulären und kugelförmigen Einfachmembranstrukturen (Liposome, Transfersome), vorliegen können. Derartige vesikuläre und kugelförmige Einfachmembranstrukturen sind jedoch in der erfindungsgemäßen Zusammensetzung unerwünscht und weitestgehend zu vermeiden.

Überraschend konnte festgestellt werden, daß die erfindungsgemäße Zusammensetzung eine hohe pharmazeutische Wirksamkeit besitzt. Diese hohe pharmazeutische Wirksamkeit der erfindungsgemäßen Zusammensetzung wird darauf zurückgeführt, daß die mit Wasser aus der Trägersubstanz ausgebildeten lamellare Doppelmembranstrukturen ähnliche physikalische Eigenschaften haben wie die Lipide der Permeabilitätsbarriere der Haut und insbesondere der lipiden Bereiche der Hornschicht. Von daher wird es verständlich, daß die erfindungsgemäße Zusammensetzung aufgrund ihrer ähnlichen oder identischen physikalischen Eigenschaften und ihrer identischen oder ähnlichen Ordnung mit der Permeabilitätsbarriere diese nicht negativ beeinträchtigen können, was sich einerseits in einem vereinfachten Eindringen und Durchdringen der erfindungsgemäßen Zusammensetzung in und durch die Hautbarrieren und andererseits in einer fehlenden Beeinträchtigung und/oder fehlenden Schädigung dieser Hautbarrieren ausdrückt. Dies wiederum hat zur Folge, daß die in der erfindungsgemäßen Zusammensetzung in einer bestimmten Struktur (lamellare Doppelmembranstruktur) vorliegende Trägersubstanz den Wirkstoff besonders schnell und gleichmäßig ohne das Hervorrufen von Hautschädigungen oder Hautirritationen und insbesondere unter Vermeidung des bei herkömmlichen Mitteln auftretenden transdermalen Wasserverlustes in bzw. durch die entsprechenden Hautbarrieren transportiert, so daß bei einer topischen Anwendung der erfindungsgemäßen Zusammensetzung insbesondere auch hiervon hervorgerufene Nebenwirkungen erheblich minimiert werden und insbesondere gar nicht erst auftreten. So konnte beispielsweise anhand von Vergleichsversuchen festgestellt werden, daß bezüglich ihres chemischen Aufbaus identische, jedoch bezüglich der Struktur der Trägersubstanz unterschiedliche Zusammensetzungen völlig unterschiedliche pharmazeutische Wirkungen und Nebenwirkungen hervorrufen. So zeigte die erfindungsgemäße, lamellare Doppelmembranstrukturen aufweisende Zusammensetzung im Vergleich zu einer liposomalen, herkömmlichen Zusammensetzung eine um 20 % bis 60 % höhere pharmazeutische Wirksamkeit und verursachte darüber hinaus selbst bei sehr empfindlichen Probanden keinerlei Hautirritationen, wobei die bekannte liposomale Zusammensetzung bei 30 % der Probanden geringe bis mittlere Hautirritationen hervorrief. Aufgrund der zuvor geschilderten höheren pharmazeutischen Wirksamkeit der erfindungsgemäßen Zusammensetzung läßt sich wiederum in der erfindungsgemäßen Zusammensetzung die Wirkstoffkonzentration im Vergleich zur herkömmlichen liposomalen Zusammensetzung reduzieren, was insbesondere dann wichtig ist, wenn toxische oder toxikologisch bedenkliche Wirkstoffe oder Wirkstoffe mit einer beträchtlichen Nebenwirkung topisch appliziert werden sollen.

Die zuvor geschilderte höhere pharmazeutische Wirksamkeit der erfindungsgemäßen Zusammensetzung führt auch dazu, daß die erfindungsgemäße Zusammensetzung insbesondere auch gut für die systemische Behandlung verwendet werden kann, da aufgrund der zuvor spezifizierten speziellen lamellaren Doppelmembranstruktur der Trägersubstanz das Eindringen der erfindungsgemäßen Zusammensetzung in die Bereiche der Epidermis, die unterhalb der Hornschicht angeordnet sind, erleichtert wird.

Ferner weist die erfindungsgemäße Zusammensetzung eine erhöhte Aufnahmekapazität sowohl für hydrophile als auch lipophile Wirkstoffe bei einer verbesserten Lagerstabilität im Vergleich zu den bekannten Zusammensetzungen auf. Dies hängt damit zusammen, daß sich die lamellaren Doppelmembranen derart zueinander ausrichten, daß sich zwischen benachbarten Doppelmembranen Wasserschichten ausbilden, die somit wasserlösliche Wirkstoffe in hohen Konzentrationen aufnehmen können, während die lipophilen Wirkstoffe unmittelbar selbst innerhalb der Doppelmembranen angeordnet sind.

Die desweiteren in der offenbarten Zusammensetzung enthaltenen Inhaltsstoffe (Verbindung mit einer funktionellen Gruppe gemäß Formel I, deren Metaboliten, S-Adenosylmethionin und N-Acyl-Ethanolamin) reduzieren desweiteren die Nebenwirkungen von Wirkstoffen oder unterdrücken diese vollständig oder wirken heilend oder präventiv, so daß die offenbarte Zusammensetzung auch solche Wirkstoffe enthalten kann, die hochtoxisch und/oder drastisch zellschädigend sind. Insbesondere fungieren das in der offenbarten Zusammensetzung vorhandene S-Adenosylmethionin und/oder die wenigstens eine funktionelle Gruppe der allgemeinen Formel I aufweisende Verbindung und/oder deren Metaboliten mittels der darin enthaltenen mindestens einen Methylgruppe als Elektronenakzeptor und fängt somit überschüssige, für den Stoffwechsel der einzelnen Zelle nicht benötigte Elektronen ab.

Diese zuvor bei der offenbarten Zusammensetzung beschriebene Wirkung ist für den Durchschnittsfachmann um so erstaunlicher, da S-Adenosylmethionin und auch Verbindungen mit der funktionellen Gruppe gemäß Formel I und auch deren Metaboliten an sich sehr stabil sind und nur unter sehr extremen thermischen Bedingungen außerhalb des Organismusses zerfallen. Hier wird angenommen, daß aufgrund der gezielten und lokalen Anwendung an der jeweils geschädigten Zelle oder den jeweils geschädigten Zellen solche Reaktionsbedingungen geschaffen werden, die die zuvor geschilderte Wirkung ermöglichen.

Der Begriff topische Anwendung deckt alle Anwendungsformen der erfindungsgemäßen Zusammensetzung ab, bei der die erfindungsgemäße Zusammensetzung über die Haut appliziert wird, wobei der Begriff Haut auch die Schleimhaut, so insbesondere im Nasen-, Mund- und Rachen-Raum oder im Vaginalbereich umfaßt. Insbesondere fallen hierunter auch solche Formulierungen, wie sie nachfolgend noch als transdermal zu verabreichende Zubereitungen beschrieben sind. Unter den Begriff N-Acyl-Ethanolamin fällt auch das N-Acylphosphatidylethanolamin.

Grundsätzlich ist festzuhalten, daß die offenbarte pharmazeutische Zusammensetzung als mit Wasser lamellare Doppelmembranstrukturen der vorstehend beschriebenen Art ausbildende Trägersubstanz solche Trägersubstanzen enthält, die einen hydrophilen sowie gleichzeitig einen hydrophoben Molekülrest aufweisen. Hier sind insbesondere bevorzugt als Substanz Monoglyceride, Diglyceride, insbesondere destillierte mittelkettige Monoglyceride, Sphingolipide, Lecithin, Phospholipide, Fettalkohole, Fettsäuren, Seifen, Mono- und/oder Di-ester von Fettsäuren, Succrose, Glucose und/oder deren Derivaten, glucosidische, furanosidische und/oder pyranosidische Kondensationsprodukte von Fettalkoholen mit Glucose und/oder Succrose und deren Polymerderivaten, Mono- und/oder Di-ester von Glucosiden mit Fettsäurederivaten, Steroide, Mono- und/oder Di-ester von Fettsäuren und Steroiden und/oder Glycol-Derivate aus Steroiden aufzuführen, wobei die Fettsäuren bevorzugt eine C₈-C₂₂ gesättigte, lineare Kohlenstoffkette besitzen.

Besonders geeignet ist es jedoch, wenn in der offenbarten pharmazeutischen Zusammensetzung als Trägersubstanz, die in der Lage ist, mit Wasser die zuvor beschriebenen lamellaren Doppelmembranstrukturen auszubilden, mindestens ein hydriertes Lecithin und/oder ein hydriertes Phospholipid, und insbesondere ein hydriertes Phosphatidylcholin, enthalten ist. Hier konnte nämlich festgestellt werden, daß derartige hydrierte Phospholipide und insbesondere das hydrierte Phosphatidylcholin einerseits im hohen Maße mit Wasser lamellare Doppelmembranstrukturen ausbildet und andererseits diese lamellaren Doppelmembranstrukturen hervorragend geeignet sind, in die interzellularen Lipide der Hornschicht zu wandern und/oder diese sogar zu durchdringen, ohne daß dadurch eine Schädigung dieser Lipidschicht eintritt.

Bevorzugt wird jedoch ein solches hydriertes Lecithin bzw. ein solches hydriertes Phospholipid und insbesondere ein solches hydriertes Phosphatidylcholin in der offenbarten Zusammensetzung vorgesehen, bei dem alle Acylreste ausschließlich oder überwiegend gesättigt sind, so daß insbesondere nur noch ungesättigte Acylreste in einer Konzentration kleiner als 10 Gew.% und vorzugsweise weniger als 5 Gew.% und ganz bevorzugt weniger als 1,5 Gew.% in dem hydrierten Lecithin, dem hydrierten Phospholipid und/oder insbesondere in dem hydrierten Phosphatidylcholin vorhanden sind.

Klarstellend sei angemerkt, daß der Begriff Phospholipid selbstverständlich nicht nur ein einzelnes Phospholipid sondern auch ein Gemisch von Phospholipiden abdeckt, wobei das Phospholipid bzw. das Phospholipidgemisch natürlichen oder synthetischen Ursprungs sein kann. Ebenso selbstverständlich ist es, daß das Phospholipid nicht im vorstehenden Sinne hydriert ist sondern daß anstelle dieses hydrierten Phospholipids ein synthetisches Phospholipid eingesetzt wird, bei dem die Acylreste im vorstehenden Sinne alle oder überwiegend gesättigt sind.

Die vorstehend beschriebenen Vorteile besitzt die erfindungsgemäße Zusammensetzung im verstärkten Maße, die als Trägersubstanz ein hydriertes Phospholipid enthält, das mindestens 60 Gew.% und vorzugsweise zwischen 70 Gew.% und 95 Gew.% hydriertes Phosphatidylcholin aufweisen, wobei sich diese Konzentrationsangaben auf die Konzentration des hydrierten Phospholipids in der anwendungsfertigen Zusammensetzung beziehen.

Bezüglich der Konzentration der in der offenbarten pharmazeutischen Zusammensetzung enthaltenen mindestens einen Trägersubstanz, die mit Wasser lamellare Doppelmembranstrukturen ausbilden kann, ist allgemein festzuhalten, daß sich diese Konzentration nach dem Speicher- und Transportvermögen der entsprechend auszubildenden lamellaren Doppelmembranstruktur für den Wirkstoff sowie für die weiteren, vorstehend genannten Inhaltsstoffe (Verbindung mit einer funktionellen Gruppe gemäß Formel I, deren Metaboliten, S-Adenosylmethionin und N-Acyl-Ethanolamin) der offenbarten Zusammensetzung richtet. Insbesondere ist diese mindestens eine Trägersubstanz in der offenbarten pharmazeutischen Zusammensetzung in einer Konzentration zwischen 0,5 Gew.% und 30 Gew.%, vorzugsweise in einer Konzentration zwischen 1 Gew.% und 15 Gew.%, vorhanden, wobei sich diese Konzentrationsangaben auf die anwendungsfertige pharmazeutische Zusammensetzung beziehen.

Insbesondere dann, wenn das zuvor beschriebene hydrierte Phospholipid, das hydrierte Phosphatidylcholin bzw. das hydrierte Lecithin bzw. ein entsprechend synthetisch hergestelltes Phospholipid mit entsprechend gesättigten Acylresten eine Phasenübergangstemperatur über 30 °C und unter 70 °C besitzt, läßt sich unter Verwendung einer derartigen Trägersubstanz besonders einfach eine Ausführungsform der offenbarten pharmazeutischen Zusammensetzung erstellen, die die erwünschten und eingangs umfangreich beschriebenen lamellaren Doppelmembranstrukturen aufweist. Hierbei ist die Phasenübergangstemperatur so definiert, daß sie die Temperatur bezeichnet, an der das kristalline System in ein flüssiges System übergeht, wobei vielfach diese Temperatur keine konkrete Einzeltemperatur sondern durch einen Temperaturbereich gekennzeichnet ist. So beträgt beispielsweise die Phasenübergangstemperatur für das besonders bevorzugte hydrierte Phosphatidylcholin, das aus Sojabohnen isoliert ist und das eine Phosphatidylcholin-Konzentration von 93 ± 3 Gew.% aufweist und dessen Acylreste zu 85 Gew.% aus Stearinsäure und zu 14 Gew.% aus Palmitinsäure bestehen, zwischen 54 °C und 58 °C, insbesondere 56 °C.

Bezüglich der in der offenbarten Zusammensetzung enthaltenen Verbindung und/oder des Metabolits ist festzuhalten, daß vorzugsweise das Metabolit bzw. die Verbindung, die mindestens eine funktionelle Gruppe der vorstehend wiedergegebenen allgemeinen Formel I enthält, ein solches Metabolit bzw. eine solche Verbindung sind, die natürlich in aeroben Zellen, insbesondere dort in den Zellmembranen, vorhanden ist. Ausdrücklich soll jedoch an dieser Stelle betont werden, daß die offenbarte Zusammensetzung als Verbindung nicht solche chemischen Komponenten aufweisen soll, die in der Technik im großen Umfang als quaternäre Ammoniumverbindungen bezeichnet werden und die synthetische grenzflächenaktive Substanzen darstellen.

Besonders vorteilhaft ist es, wenn die offenbarte Zusammensetzung als Verbindung Betain, Acetylcholin, Cholin, Glycerophosphocholin, Phosphatidylcholin, Lysophosphatidylcholin, Carnitin, Acylcarnitin, Sphingomyeline jeweils allein oder in Mischung untereinander und/oder Derivate und/oder Metabolite hiervon enthält.

Im Zusammenhang mit der offenbarten pharmazeutischen Zusammensetzung ist festzuhalten, daß diese wahlweise oder additiv auch mindestens ein Metabolit der in der offenbarten Zusammensetzung enthaltenen Verbindung mit der in Formel I wiedergegebenen funktionellen Gruppe aufweisen kann. Bevorzugte Metaboliten dieser Verbindung sind insbesondere Methylglycin, Dimethylglycin und/oder Methylmethionin, so daß dementsprechend besonders geeignete Ausführungsformen der offenbarten pharmazeutischen Zusammensetzung dann als Metabolit Methylglycin, Dimethylglycin und/oder Methylmethionin, jeweils allein oder in Mischung, enthalten können.

Abhängig vom jeweiligen Anwendungszweck richtet sich bei der offenbarten Zusammensetzung die Konzentration der in der offenbarten Zusammensetzung enthaltenen Verbindung mit der in Formel I wiedergegebenen funktionellen Gruppe und/oder deren Metaboliten und/oder die Konzentration des S-Adenosylmethionins. Besonders bevorzugt ist es, wenn die Verbindung und/oder deren Metaboliten und/oder das S-Adenosylmethionin in einer Konzentration zwischen 0,0001 % und 10 %, vorzugsweise zwischen 0,1 % und 9 %, jeweils bezogen auf das Gewicht der anwendungsfertigen pharmazeutischen Zusammensetzung, vorhanden ist.

Eine mit einer besonders hohen pharmazeutischen Wirksamkeit und mit geringen Nebenwirkungen versehene Ausgestaltung der erfindungsgemäßen pharmazeutische Zusammensetzung sieht vor, daß hierbei die Zusammensetzung eine Mischung enthält, die als Inhaltsstoffe Betain, Methylglycin und/oder N-Acyl-Ethanolamin umfaßt. Hierbei kann diese Ausgestaltung wahlweise entweder alle zuvor genannten drei bevorzugten Inhaltsstoffe aufweisen oder nur zwei Inhaltsstoffe der zuvor genannten Art enthalten, so insbesondere die Kombination von Betain und Methylglycin, die Kombination von N-Acyl-Ethanolamin und Methylglycin, die Kombination von Betain und N-Acyl-Ethanolamin sowie die Kombination von Betain mit N-Acyl-Ethanolamin und Methylglycin.

Weist die zuvor beschriebene Ausgestaltung der erfindungsgemäßen pharmazeutischen Zusammensetzung eine Mischung aus zwei der zuvor genannten speziellen Inhaltsstoffe auf, so variiert das Mol-Massen-Verhältnis dieser beiden Inhaltsstoffe (Betain, Methylglycin, N-Acyl-Ethanolamin) insbesondere zwischen 1:1 bis 1:9.

Der in der vorliegenden erfindungsgemäßen Zusammensetzung enthaltene mindestens eine Wirkstoff umfaßt grundsätzlich die Gruppe aller Wirkstoffe, die topisch anwendbar sind. Insbesondere weist jedoch die erfindungsgemäße Zusammensetzung einen solchen Wirkstoff auf, der aus der Gruppe ausgewählt ist, die Analgetika, Antirheumatika, Antiallergika, Antibiotika, Antimykotika, Antiphlogistika, Balneotherapeutika, corticoide Wirkstoffe, Dermatika, Antiseptika, durchblutungsfördernde Wirkstoffe, Sedativa, Anästhetika, Spasmolytika, Vitamine und Wundbehandlungsmittels jeweils allein oder in Mischung umfaßt.

Insbesondere weist jedoch die erfindungsgemäße pharmazeutische Zusammensetzung in ihrer bevorzugten Ausführungsform einen Wirkstoff auf, der ein lokal wirkendes Dermatikum darstellt, wobei in dieser bevorzugten Ausführungsform sowohl ein einzelnes Dermatikum als auch eine Mischung von verschiedenen Dermatika vorgesehen sein kann. Hierbei wird diese bevorzugte Ausführungsform insbesondere zur Behandlung von Akne, Furunkeln, Ekzemen, infizierten Hauterkrankungen, so insbesondere Pilzerkrankungen, Brandwunden, Dekubitus, Pickeln, Pusteln, nekrotischen Geweben, Abszessen, Schürfwunden, Sonnenbrand, Insektenstichen, entzündlichen Hauterkrankungen und Hautreizungen und/oder Ödemen verwendet und weist insbesondere einen Wirkstoff bzw. eine Wirkstoffmischung auf, die aus der Gruppe, umfassend Tetracyclin, Erythromycin, Chlortetracyclin, Framycetinsulfat, Fusidinsäure, Fusidinsäuresalze, Chloramphenicol, Clindamycin, Gentamicinsulfat, Neomycin, Cortison und Cortisonderivate, insbesondere Progesteron, Testosteron, Aldosteron, Hydrocortison und/oder Cortisol, Chlorokresol, Econazol, Miconazol, Thioconazol, Griseofulvin, Clotrimazol, Lidocain, Ketocain, Prilocain, NSAID, Indomethacin, Ibuprofen, Sulindac, Naproxen, Ketoprofen, Proxicam und/oder Diclofenac ausgewählt ist.

Insbesondere variiert die Konzentration der zuvor aufgeführten und in der erfindungsgemäßen Zusammensetzung enthaltenen Wirkstoffe zwischen 0,05 % und 20 %, vorzugsweise zwischen 0,1 % und 4 %, bezogen auf das Gewicht der anwendungsfertigen pharmazeutischen Zusammensetzung.

Bei einer besonders vorteilhaften Weiterbildung der zuvor beschriebenen Ausführungsformen der erfindungsgemäßen Zusammensetzung weist diese Weiterbildung als Verbindung eine Fettsäure und/oder ein Fettsäuresalz des Betains und/oder eine Mischung aus Betain mit mindestens einer Fettsäure und/oder eine Mischung aus Betain mit mindestens einem Fettsäuresalz auf.

Vorzugsweise wird hierbei als Fettsäuresalz ein solches Salz ausgewählt, bei dem die zugrundeliegende Fettsäure eine lineare Fettsäure ist und zwischen 12 bis 22 Kohlenstoffatomen aufweist, während bei Verwendung einer Fettsäure diese vorzugsweise ebenfalls zwischen 12 und 22 Kohlenstoffatomen enthält.

Besonderes geeignete Fettsäuresalze des Betains stellen Betainlaurat, Betainmyristat, Betainpalmitat, Betainstearat, Betainoleat und Betainlinolat, jeweils allein oder in Mischung, dar. Hier konnte überraschend festgestellt werden, daß insbesondere diese zuvor konkret genannten Fettsäuresalze des Betains trotz ihrer relativ schlechten Wasserlöslichkeit dazu beitragen, daß die erfindungsgemäße pharmazeutische Zusammensetzung eine besonders hohe pharmazeutische Wirksamkeit besitzt.

Wie bereits eingangs ausgeführt ist, weist die erfindungsgemäßen pharmazeutische Zusammensetzung Wasser auf, wobei die Konzentration des Wassers in der erfindungsgemäßen Zusammensetzung insbesondere zwischen 5 % und 90 %, bezogen auf das Gewicht der anwendungsfertigen pharmazeutischen Zusammensetzung, variiert.

Der in der vorliegenden Anmeldung verwendete Begriff Wasser deckt alle wäßrigen Systeme, so insbesondere sterilisiertes Wasser, entionisiertes Wasser, destilliertes Wasser sowie wäßrige Lösungen und/oder wäßrige Puffersysteme, ab.

Desweiteren können, je nach der Art der jeweils gewählten Formulierung, in der erfindungsgemäßen pharmazeutischen Zusammensetzung mindestens ein Konservierungsmittel, ein Antioxidants, ein Verdickungsmittel, ein Gelbildner, eine osmoaktive Substanz, vorzugsweise Prolin, 4-Hydroxy-Prolin, und/oder Inositol, einen sonstigen Zusatzstoff, insbesondere ein Calciumchlorid und/oder mindestens ein Alkohol, vorzugsweise ein mehrwertiger Alkohol, enthalten sein. Hierbei variiert Konzentration der zuvor genannten osmoaktiven Substanz insbesondere zwischen 0,02 Gew.% und 30 Gew.%, bezogen auf die anwendungsfertige Zusammensetzung.

Vorzugsweise ist zu der Konzentration des in der erfindungsgemäßen pharmazeutischen Zusammensetzung enthaltenen N-Acyl-Ethanolamins festzuhalten, daß diese Konzentration zwischen 0,01 % und 10 %, insbesondere zwischen 0,1 % und 3 %, jeweils bezogen auf das Gewicht der anwendungsfertigen pharmazeutischen Zusammensetzung, variiert.

Wiederholt wurde vorstehend im Zusammenhang mit der erfindungsgemäßen pharmazeutischen Zusammensetzung ausgeführt, daß in der erfindungsgemäßen pharmazeutischen Zusammensetzung ein N-Acyl-Ethanolamin enthalten ist. Insbesondere weist dieses N-Acyl-Ethanolamin einen C₁-C₁₈-Acylrest, vorzugsweise einen linearen gesättigten und/oder ungesättigten C₁-C₁₈-Acylrest auf.

Eine bevorzugte Ausführungsform der erfindungsgemäßen pharmazeutischen Zusammensetzung greift auf ein N-Acyl-Ethanolamin oder eine Mischung von N-Acyl-Ethanolamine zurück, die aus der Gruppe ausgewählt sind, die Lactamid MEA, Oleamid MEA, Acetamid MEA, N-Acetyl-Phosphatidylethanolamin, N-Acetyl-Ethanolamin, N-Oleoyl-Ethanolamin, N-Linolenoyl-Ethanolamin, N-Cocoyl-Ethanolamin, N-Palmitinoyl-Ethanolamin sowie N-Acyl-2-Hydroxy-Propylamin umfaßt, wobei die zuletzt genannte Verbindung dann insbesondere als Acylrest Fettsäuren aus Kokosfett und/oder Palmöl enthält.

Eine besonders bevorzugte und vielfältig anzuwendende Ausgestaltung der offenbarten Zusammensetzung enthält zwischen
5 % und 90 % Wasser,
1 % und 15 % Trägersubstanz,
0,0001 % bis 10 % der Verbindung und/oder des Metaboliten und/oder des S-Adenosylmethionins,
0,05 % bis 20 % des mindestens einen Wirkstoffes,
0,01 % und 10 % des N-Acyl-Ethanolamins
sowie übliche sonstige, und insbesondere vorstehend genannte Bestandteile (Konservierungsmittel, Antioxidantien, Verdickungsmittel, Gelbildner, osmoaktive Substanz und/oder einen Alkohol, vorzugsweise einen mehrwertigen Alkohol) in einer Konzentration zwischen 0 % und 60 %
enthält, wobei sich die zuvor wiedergegebenen Konzentrationsangaben auf das Gewicht der anwendungsfertigen Zusammensetzung beziehen.

Die erfindungsgemäße pharmazeutische Zusammensetzung kann grundsätzlich in jeder, zur topischen Anwendung geeigneten Formulierung aufgemacht sein, wobei insbesondere die erfindungsgemäßen pharmazeutische Zusammensetzung als topisch applizierbares Gel formuliert ist. Hierbei weist dieses topisch applizierbare Gel eine Viskosität bei 20 °C zwischen 4.000 mPas und 40.000 mPas, vorzugsweise zwischen 12.000 mPas und 25.000 mPas, auf, so daß sich ein derartig formuliertes Gel einwandfrei und besonders glatt auf der Haut verteilen läßt.

Um die Anwendung der erfindungsgemäßen pharmazeutischen Zusammensetzung insbesondere auch bei Probanden mit empfindlicher Haut sicherzustellen, weist vorzugsweise die erfindungsgemäße Zusammensetzung einen solchen pH-Wert auf, der zwischen 4,0 und 7,6 variiert.

Wie bereits eingangs bei der erfindungsgemäßen Zusammensetzung ausführlich erläutert ist, wird als eine Ursache für die verbesserte pharmazeutische Wirksamkeit und die Minimierung und/oder die Eliminierung der Nebenwirkungen der erfindungsgemäßen Zusammensetzung angenommen, daß in der erfindungsgemäßen Zusammensetzung mindestens eine solche Trägersubstanz enthalten ist, die lamellare Doppelmembranstrukturen ausbildet. Insbesondere dann, wenn die erfindungsgemäße Zusammensetzung zwischen 15 Gew. % und 95 Gew.%, vorzugsweise zwischen 30 Gew.% und 95 Gew.%, derartiger lamellarer Doppelmembranstrukturen enthält, wobei sich die zuvor angegebenen Konzentrationen auf das Gewicht der in der erfindungsgemäßen Zusammensetzung enthaltenen Trägersubstanz bezieht, weist eine derartige Ausgestaltung eine besonders hohe pharmazeutische Wirksamkeit auf, da aufgrund der hohen Konzentration an lamellaren Doppelmembranstrukturen die hierin integrierten Inhaltsstoffe (Verbindung mit einer funktionellen Gruppe gemäß Formel I, deren Metaboliten, S-Adenosylmethionin und N-Acyl-Ethanolamin), in besonders hohen Konzentrationen und besonders schnell in die interzellularen Lipide der Hornschicht und ggf. hierdurch transportiert werden, so daß dort die eingangs bei der erfindungsgemäßen Zusammensetzung geschilderten Vorteile im verstärkten Maße bei dieser Ausführungsform vorhanden sind.

Vorzugsweise weist die erfindungsgemäße Zusammensetzung eine solche lamellare Doppelmembranstruktur auf, bei der jede einzelne Doppelmembran eine Dicke zwischen 4 nm und 20 nm, vorzugsweise zwischen 4 nm und 8 nm, besitzt.

Eine weitere, besondere bevorzugte Ausführungsform der erfindungsgemäßen Zusammensetzung sieht vor, daß die erfindungsgemäße Zusammensetzung, die in den zuvor beschriebenen Ausführungsformen vorliegen kann, als Wirkstoff ein Vitamin, vorzugsweise ein Vitamin E, allein oder in Mischung mit weiteren Vitaminen aufweisen kann. Hier hat sich überraschend gezeigt, daß eine derartige, Vitamin-enthaltende Zusammensetzung hervorragend geeignet ist, um Nebenwirkungen auf der Haut, so insbesondere Hautreizungen, Pustel- und Pickelbildung, allergische Hautreaktionen und/oder Hautentzündungen, bei der oralen und/oder injizierten Anwendung von bestimmten Medikamenten, wie beispielsweise bei der Gabe von Penicillinen, Empfängnisverhütungsmitteln, Cytostatika, Sulfonamide und/oder Barbiturate, wirksam zu verhindern oder schnell einer Heilung zuzuführen. Hier hat sich gezeigt, daß bei Auftreten dieser zuvor angesprochenen Hautnebenwirkungen bereits eine auf wenige Anwendungen begrenzte topische Behandlung der betroffenen Hautpartien mit der zuvor beschriebenen Ausführungsform der erfindungsgemäßen Zusammensetzung ausreicht, um diese Nebenwirkungen zu beseitigen, wobei auch eine gewisse Heilung dann zu erreichen ist, wenn die zuvor beschriebene Ausführungsform der erfindungsgemäßen Zusammensetzung keinen Wirkstoff und somit nur die Trägersubstanz, Wasser und eine Mischung insbesondere aus
a) Cholin, Betain, Carnitin, Methylmethionin und/oder Phosphatidylcholin,
b) N-Acyl-Ethanolamin mit einem C₁₀-C₁₈-Acylrest und
c) Methylglycin
in den nachfolgend noch bei der erfindungsgemäßen transdermal zu verabreichenden Zubereitung angegebenen Massenverhältnissen enthält. Eine ebenfalls hierfür geeignete Ausführungsform der erfindungsgemäßen Zusammensetzung weist an Stelle des Vitamins oder zusätzlich zu dem Vitamin als Wirkstoff Dimethylselfoxid auf.

Wie bereits vorstehend dargelegt wurde, wird die erfindungsgemäße pharmazeutische Zusammensetzung insbesondere topisch appliziert, wobei eine bevorzugte Ausführungsform dieser Applikation dann eine transdermal zu verabreichende Zubereitung betrifft, die nachfolgend auch kurz als erfindungsgemäße Zubereitung bezeichnet wird und die als Wirkstoffreservoir eine pharmazeutische Zusammensetzung enthält, wie diese vorstehend umfangreich beschrieben ist. Von daher gelten für die erfindungsgemäß Zubereitung alle die Aussagen, wie sie vorstehend für die erfindungsgemäße Zusammensetzung und deren diversen Weiterbildungen umfangreich beschrieben sind, so daß zur Vermeidung von Wiederholungen auf die vorstehenden Aussagen, die dort beschriebenen Parameter sowie die besonders hervorgehobenen Vorteile verwiesen wird. Hierbei bedeutet der Begriff transdermal, daß die erfindungsgemäße Zubereitung auf einen Bereich der Haut, so zum Beispiel auf einen Hautbereich des Rückens, der Arme und/oder der Beine aufgetragen wird, um so zu erreichen, daß über einen vorgegebenen Zeitraum der mindestens in der erfindungsgemäßen Zusammensetzung enthaltene eine Wirkstoff in die entsprechenden Hautschichten und/oder penetriert und dort das jeweilige Wirkstoffspektrum prophylaktisch und/oder therapeutisch entfaltet und/oder daß der jeweilige Wirkstoff die Haut permeatiert, so daß der Wirkstoff von den unterhalb der Haut vorgesehenen Blut-oder Lymphgefäße resorbiert werden kann.

Ein wesentlicher Vorteil der erfindungsgemäß transdermal zu verabreichenden Zubereitung ist darin begründet, daß die erfindungsgemäße Zubereitung die zuvor beschriebene Zusammensetzung als Wirkstoffreservoir enthält, wobei aufgrund der zuvor bei der erfindungsgemäßen Zusammensetzung beschriebenen Trägersubstanz, die eine lamellare Doppelmembranstruktur aufweist, einerseits eine hervorragende Hautverträglichkeit aufgrund ihrer Ähnlichkeit mit der Haut und insbesondere aufgrund ihrer Ähnlichkeit mit den Lipidbereichen der Hornschicht besitzt und andererseits sicherstellt, daß der jeweilige Wirkstoff besonders gut und gleichmäßig in die Haut eindringt (penetriert) und/oder den Hautdurchtritt besonders reproduzierbar bewirkt (permeatiert), so daß dementsprechend reproduzierbare Mengen des jeweiligen Wirkstoffes über einen vorgegebenen Zeitraum von den daruntar angeordneten Blut- und/oder Lymphgefäßen aufgenommen, d.h. somit resorbiert, wird, ohne daß bei dieser Anwendung Nebenwirkungen, wie beispielsweise Hautreizungen, Rötungen, Hautbrennen oder sonstige Hautbeeinflussungen und/oder Hautschädigungen, auftreten.

Eine besonders geeignete Ausgestaltung der zuvor beschriebenen erfindungsgemäßen transdermal zu verabreichenden Zubereitung sieht vor, daß hierbei die erfindungsgemäße Zubereitung eine solche pharmazeutische Zusammensetzung enthält, die neben mindestens einer Trägersubstanz, wie sie zuvor bei der erfindungsgemäßen Zusammensetzung umfangreich beschrieben ist, und Wasser desweiteren
a) Cholin, Betain, Carnitin, Methylmethionin und/oder Phosphatidylcholin,
b) N-Acyl-Ethanolamin mit einem C₁₀-C₁₈-Acylrest,
c) Methylglycin sowie
d) mindestens einen transdermal zu verabreichenden, in die Haut penetrierenden und/oder die Haut permeatierenden Wirkstoff
aufweist.

Bezüglich des Wirkstoffes bei dieser Ausführungsform der erfindungsgemäßen Zubereitung gelten die Aussagen, wie sie vorstehend für den Wirkstoff bei der erfindungsgemäßen Zusammensetzung bereits dargelegt wurden.

Als besonders geeignete Wirkstoffe, die über die erfindungsgemäße Zubereitung transdermal verabreicht werden und die insbesondere auch systemisch wirken, sind die bei der Nitrattherapie bei Angina pectoris verwendeten Wirkstoff Glyceroltrinitrat, Isosorbidditrat und/oder Pentraerythryltetranitrat, das bei Kinetose anzuwendende L-Hyoscin, das zur Nikotin-Entwöhnung angewandte Nicotin und dessen Derivate, das zur Schmerz- und Entzündungsbehandlung verwendete Fentanyl, Flurbiprofen, Ketoprofen oder Diclofenac, insbesondere Diclofenac-Hydroxyethylpyrrolidin, das zur Blutdrucksenkung eingesetzte Clonidin und/oder die bei der Hormonsubstitution verwendeten Hormone, insbesondere Estradiol, Testosteron und/oder Mischungen aus Estradiol und Norethisteronacetat, zu nennen.

Bezüglich der Massenverhältnisse in der erfindungsgemäßen transdermal zu verabreichende Zubereitung enthaltenen und vorstehend aufgeführten Komponente a) (Cholin, Betain, Carnitin, Methylmethionin und/oder Phosphatidylcholin) zur Komponente b) (N-Acyl-Ethanolamin mit einem C₁₀-C₁₈-Acylrest) und zur Komponente c) (Methylglycin) ist festzuhalten, daß diese Massenverhältnisse vorzugsweise zwischen 1:1:1 und 1:10:1 oder zwischen 1:1:1 und 10:1:1 oder zwischen 1:1:1 und 1:1:10 variiert, wobei sich die zuvor angegebenen Massenverhältnisse insbesondere auch danach richten, welche Trägersubstanz und welchen Wirkstoff in der erfindungsgemäßen Zubereitung enthalten sind und ob die erfindungsgemäße Zubereitung nur die Haut penetrieren oder auch die Haut permeatieren soll.

Insbesondere dann, wenn die erfindungsgemäße transdermal zu verabreichende Zubereitung die Haut permeatieren soll, weist diese Ausführungsform der erfindungsgemäßen Zubereitung einen solche Wirkstoff auf, der ein systemisch wirkender Wirkstoff ist. Beispiele für systemisch wirkende Wirkstoffe sind bereits zuvor bei der erfindungsgemäßen Zusammensetzung auch umfangreich beschrieben worden.

Im einfachsten Fall wird die erfindungsgemäße, transdermal zu verabreichende Zubereitung auf die jeweilig ausgewählte Hautzone in einer vorgegebenen Menge aufgetragen und dort kurz einmassiert oder eingerieben, wobei entsprechende Dosiervorrichtungen bekannt sind, um entsprechende, exakt eingestellte Mengen einer insbesondere pastösen oder gelartigen Zubereitung genau und reproduzierbar auf ausgewählte Hautzonen aufzutragen. Hier hat sich überraschend gezeigt, daß auch ohne zusätzliche Abdeckung, beispielsweise in Form eines entsprechenden äußeren Schutzelementes, reproduzierbare Mengen an Wirkstoff aus der als Wirkstoffreservoir dienenden erfindungsgemäßen pharmazeutischen Zusammensetzung freigesetzt werden, selbst dann, wenn die nicht abgedeckte Zubereitung mit Wasser, beispielsweise im Rahmen der Körperhygiene, in Kontakt kommt.

Um jedoch auch ohne zusätzliches Einreiben bzw. Einmassieren der erfindungsgemäßen Zubereitung sicherzustellen, daß die erfindungsgemäße Zubereitung dauerhaft den Wirkstoff aus der als Wirkstoffreservoir dienenden erfindungsgemäßen pharmazeutischen Zusammensetzung freisetzt, sieht eine weitere Ausführungsform der erfindungsgemäßen transdermal zu verabreichenden Zubereitung vor, daß hierbei die Zubereitung noch ein das Wirkstoffreservoir abdeckendes Schutzelement umfaßt. Hierbei kann es sich beispielsweise um eine übliche Abdeckfolie handeln, die als Folie oder in Form eines Sprays auf das Wirkstoffreservoir aufgebracht wird und so das Wirkstoffreservoir gegen äußere Einflüsse schützt. Ebenso gut kann die erfindungsgemäße Zubereitung als herkömmliches Pflaster ausgebildet sein, wie dieses für transdermale therapeutische Systeme bekannt und üblich ist.

Inositol, wie es vorstehend und nachfolgend genannt ist, ist chemisch korrekt als Inosite (Cyclohexan-1,2,3,4,5,6-hexaole) zu bezeichnen.

Insbesondere wird als Methylmethionin in der erfindungsgemäßen Zusammensetzung ein Salz von Methylmethionin und vorzugsweise S-Methyl-DL-Methionin-Sulfoniumchlorid verwendet.

Vorteilhafte Weiterbildungen der erfindungsgemäßen Zusammensetzung sind in den Unteransprüchen angegeben.

Die erfindungsgemäße Zusammensetzung wird nachfolgend anhand von fünf Ausführungsbeispielen und in Verbindung mit der Zeichnung näher erläutert. Es zeigen:
- Figur 1: schematisch eine einzige Doppelmembranstruktur und
- Figur 2: schematisch eine Struktur von drei sandwichartig angeordneten Doppelmembranen

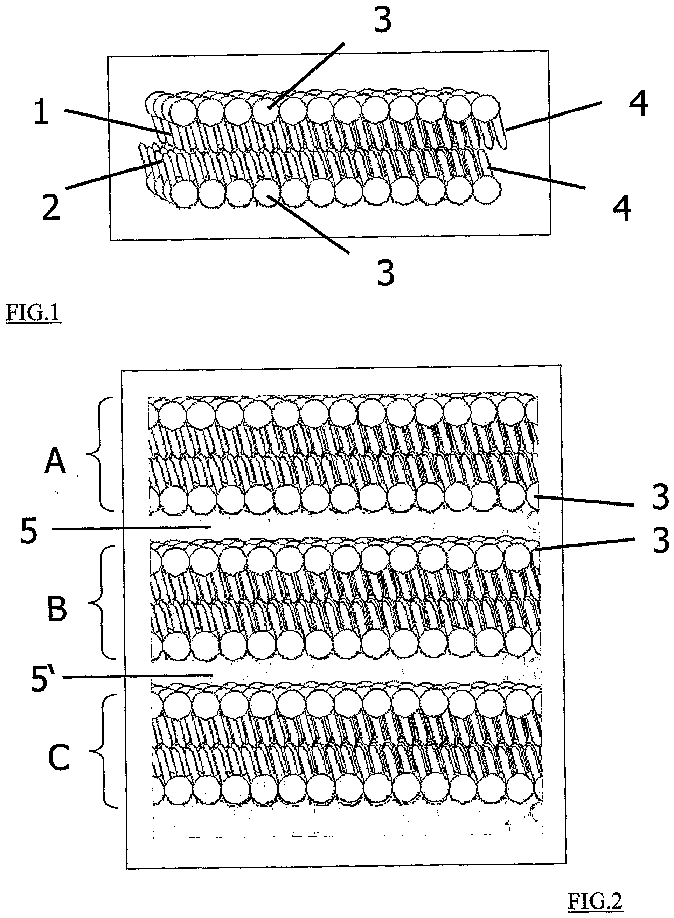

In den Figuren 1 und 2 sind jeweils lamellare Doppelmembranstrukturen schematisch abgebildet, die die Trägersubstanz in Verbindung mit Wasser ausbildet.

Wie den beiden Figuren 1 und 2 zu entnehmen ist, besitzt jede lamellare Doppelmembranstruktur einen Schichtaufbau, derart, daß eine erste Trägersubstanz-Schicht 1 parallel zu einer zweiten Trägersubstanz-Schicht 2 ausgerichtet ist, wobei die hydrophilen Reste 3 einer jeden Trägersubstanz-Schicht 1 bzw. 2 jeweils nach außen und somit zu der die lamellare Doppelmembranstruktur umgebene wäßrige Schicht ausgerichtet sind. Dementsprechend sind die lipophilen Reste 4 einer jeden lamellaren Doppelmembranstruktur nach innen hin positioniert.

Während die zuvor beschriebene Figur 1 eine einzelne Doppelmembranstruktur abbildet, sind in der Figur 2 schematisch drei Doppelmembranstrukturen, die vorstehend auch als Doppelmembrane bezeichnet sind, abgebildet. Hierbei besteht die in Figur 2 schematisch gezeigte Struktur aus drei, sandwichartig übereinander angeordnete Doppelmembranen A, B und C, wobei jede Doppelmembran A, B und C einen solchen Aufbau aufweist, wie dieser vorstehend für die in Figur 1 gezeigte Doppelmembran beschrieben wurde. Zwischen jeder Doppelmembran A, B und C ist jeweils mindestens eine Wasserschicht 5 bzw. 5' vorgesehen, die die lipophilen Reste 3 einer jeder Doppelmembran auf Abstand hält, wie dies beispielsweise in Figur 2 für die Doppelmembran A und die Doppelmembran B gezeigt ist.

### Ausführungsbeispiel 1

### entzündungshemmende topisch anzuwendende gelartige Zusammensetzung

Es wird eine pharmazeutische Zusammensetzung aus den nachfolgend aufgelisteten Inhaltsstoffen erstellt:

**Phase 1**

| | |
|---|---|
| hydriertes Phosphatidylcholin, Konzentration an hydriertem Phosphatidylcholin 90 Gew.% | 1,95 % |
| Palmitinsäure | 0,8 % |
| Cetearylalkohol | 0,3 % |
| Capryl-/Caprinsäure-Triglyceride | 16,0 % |
| Diclofenac | 4,0 % |
| Ethanol | 10,0 % |

**Phase 2**

| | |
|---|---|
| Propylenglykol | 6,0 % |
| Glycerin | 5,0 % |
| Acetamid MEA | 0,5 % |
| Betain, wasserfrei | 0,3 % |
| Sarcosin | 0,7 % |
| Wasser DAB 10 | ad 100,0 % |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 20.000 U/min homogenisiert.

An diese Homogenisierung schloß sich eine acht Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 14.000 U/min. Unter kontinuierlichem Rühren wurde danach die Mischung auf Raumtemperatur abgekühlt.

### Ausführungsbeispiel 2

### cortisonhaltige, entzündungshemmende, topisch anzuwendende gelartige Zusammensetzung

Es wird eine pharmazeutische Zusammensetzung aus den nachfolgend aufgelisteten Inhaltsstoffen erstellt:

**Phase 1**

| | |
|---|---|
| hydriertes Phosphatidylcholin, Konzentration an hydriertem Phosphatidylcholin 90 Gew.% | 1,95 % |
| C12-C16-Alkylbenzoate | 16,0 % |
| Palmitinsäure | 0,8 % |
| Cetearylalkohol | 0,3 % |
| Hydrocortison | 1,0 % |
| Ethanol | 10,0 % |

**Phase 2**

| | |
|---|---|
| Propylenglykol | 6,0 % |
| Glycerin | 5,0 % |
| Acetamid MEA | 0,5 % |
| Betain, wasserfrei | 0,3 % |
| Sarcosin | 0,7 % |
| Calciumchlorid | 0,3 % |
| Wasser DAB 10 | ad 100,0 % |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 20.000 U/min homogenisiert.

An diese Homogenisierung schloß sich eine acht Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 14.000 U/min. Unter kontinuierlichem Rühren wurde danach die Mischung auf Raumtemperatur abgekühlt.

### Ausführungsbeispiel 3

### antimykotisch und antibakteriell, topisch anzuwendende gelartige Zusammensetzung

Es wird eine pharmazeutische Zusammensetzung aus den nachfolgend aufgelisteten Inhaltsstoffen erstellt:

**Phase 1**

| | |
|---|---|
| hydriertes Phosphatidylcholin, Konzentration an hydriertem Phosphatidylcholin 90 Gew.% | 2,1 % |
| Stearinsäure | 1,0 % |
| Cetearylalkohol | 0,5 % |
| C12-C16-Alkylbenzoate | 16,0 % |
| Clotrimazol | 1,0 % |
| Ethanol | 10,0 % |
| Cocoamid MEA | 0,2 % |

**Phase 2**

| | |
|---|---|
| Propylenglykol | 6,0 % |
| Glycerin | 5,0 % |
| Betain, wasserfrei | 0,3 % |
| Sarcosin | 0,2 % |
| Wasser DAB 10 | ad 100,0 % |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 18.000 U/min homogenisiert.

An diese Homogenisierung schloß sich eine sechs Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 10.000 U/min. Unter kontinuierlichem Rühren wurde danach die Mischung auf Raumtemperatur abgekühlt.

Die in Ausführungsbeispiel 3 beschriebene Zusammensetzung, die nachfolgend auch kurz als Zusammensetzung 3 bezeichnet wird, wurde in einer ersten Studie im Vergleich zu einem herkömmlichen, im Handel erhältlichen cremeartigen Mittel zur Behandlung von Fußmykosen erprobt. Hierbei umfaßte diese Studie 30 Probanden, die jeweils an beiden Füßen an Fußmykose litten.

Teilweise waren die mit Fußmykose befallenen Bereiche, insbesondere zwischen den Zehen, stark nässend und offen, was aufgrund des Juckreizes und Kratzens der Probanden erklärlich war.

Täglich einmal wurde auf einem Fuß der Probanden die Zusammensetzung 3 und auf dem anderen Fuß das herkömmliche cremeartige Mittel aufgetragen.

Nach einer Behandlungszeit jeweils von einer Woche, nach zwei Wochen und nach drei Wochen wurde der jeweils behandelte Fuß auf Pilzelemente untersucht, wobei das Ergebnis dieser Untersuchung in der nachfolgenden Tabelle 1 wiedergegeben ist.

**Tabelle 1**

| Anzahl der Probanden ohne Pilzelemente an dem jeweils behandelten Fuß nach jeweils 1 Woche, 2 Wochen und 3 Wochen bei einem einmaligen Auftragen pro Tag | | |
|---|---|---|
| | Anzahl der Probanden | |
| Behandlungszeit | Fuß behandelt mit Zusammensetzung 3 | Fuß behandelt mit herkömmlichen Mittel |
| 1 Woche | 17 | 8 |
| 2 Wochen | 23 | 14 |
| 3 Wochen | 29 | 22 |

Die vorstehende Tabelle 1 belegt die deutliche Überlegenheit der Zusammensetzung 3 im Vergleich zu dem herkömmlichen Mittel. Übereinstimmend berichteten alle Probanden, insbesondere auch die Probanden, die nässende, teilweise offene Pilzerkrankungen zeigten, daß bereits das einmalige Auftragen der Zusammensetzung 3 eine sofortige Linderung der durch die offenen und nässenden Bereiche hervorgerufenen Schmerzen ergab, während die mit dem herkömmlichen Mittel behandelten Patienten teilweise über zusätzliche Schmerzen klagten. Insbesondere konnten auch bei den mit der Zusammensetzung 3 behandelten Patienten keine Hautrötungen festgestellt werden, was bei vier, offensichtlich empfindlichen Patienten, die mit dem herkömmlichen Mittel behandelt wurden, auftrat.

### Ausführungsbeispiel 4

### lokalanästhetisch, topisch anzuwendende gelartige Zusammensetzung

Es wird eine pharmazeutische Zusammensetzung aus den nachfolgend aufgelisteten Inhaltsstoffen erstellt:

**Phase 1**

| | |
|---|---|
| hydriertes Phosphatidylcholin, Konzentration anhydriertem Phosphatidylcholin 90 Gew.% | 5,0 % |
| Palmitinsäure | 1,0 % |
| Cetearylalkohol | 0,5 % |
| Isopropylpalmitat | 16,0 % |
| Lidocain | 1,0 % |
| Sodium Carbomer | 0,25 % |
| Ethanol | 10,0 % |

**Phase 2**

| | |
|---|---|
| Propylenglykol | 6,0 % |
| Glycerin | 5,0 % |
| Lactamid MEA | 1,0 % |
| Betain, wasserfrei. | 0,3 % |
| Sarcosin | 0,2 % |
| Wasser DAB 10 | ad 100,0 % |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 15.000 U/min homogenisiert.

An diese Homogenisierung schloß sich eine sechs Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 10.000 U/min. Unter kontinuierlichem Rühren wurde danach die Mischung auf Raumtemperatur abgekühlt.

Die vorstehend nach Ausführungsbeispiel 4 hergestellte lokal anästhetisch wirkende topische Zusammensetzung wurde wie folgt erprobt.

20 erwachsene Probanden, jeweils 10 männliche und 10 weibliche Probanden, wurden mit einer Zusammensetzung gemäß Ausführungsbeispiel 4, die nachfolgend auch kurz Zusammensetzung 4 bezeichnet ist, und einer weiteren Zusammensetzung, die mit Ausnahme des bei Beispiel 4 genannten Lidocains, das ersatzlos gestrichen wurde, behandelt, wobei diese Zusammensetzung ohne Lidocain nachfolgend auch kurz als Zusammensetzung 4 B bezeichnet wird.

Hierbei wurden identischen Mengen von jeder Zusammensetzung, d.h. der Zusammensetzung 4 und der Zusammensetzung 4 B, auf jeweils eine 12 cm² große, zuvor gekennzeichnete Hautfläche eines jeden Probanden auf den Rücken in einer gleichmäßigen Schichtdicke aufgetragen. Nach einer Verweilzeit von jeweils 30 Minuten, 1 Stunde, 2 Stunden und 4 Stunden nach Auftragen der Zusammensetzung 4 bzw. 4 B wurde die jeweils behandelte Hautzone dahingehend getestet, daß in jeder, 12 cm² großen Hautzone jeweils 10 Stiche mit einer sterilisierten Stecknadel bei einer Eindringtiefe von 3 mm vorgenommen wurde.

Das subjektive Schmerzempfinden der Probanden wurde ermittelt.

Als Ergebnis dieser Probandenstudie ist festzuhalten, daß alle Probanden übereinstimmend berichteten, daß die mit der Zusammensetzung 4 B behandelten Hautzonen bei jedem Einstich mit der Nadel schmerzhaft waren.

Bezüglich des Einstichschmerzes bei der mit der Zusammensetzung 4 behandelten Hautzonen ergab sich das in Tabelle 1 wiedergegebene Ergebnis, wobei zu jeder Verweilzeit nach Applikation der Zusammensetzungen 4 und 4 B bei allen Probanden insgesamt 200 Einstiche (20 Probanden jeweils 10 Einstiche) vorgenommen wurden.

**Tabelle 2**

| *Anzahl der schmerzunempfindlichen und schmerzempfindlichen Ein stiche, jeweils nach einer Verweilzeit von 30 Minuten, 1 Stunde, 2 Stunden und 4 Stunden* | | |
|---|---|---|
| Verweilzeit | schmerzunempfindliche Einstiche | schmerzempfindliche Einstiche |
| 30 Minuten | 185 | 15 |
| 1 Stunde | 195 | 5 |
| 2 Stunden | 195 | 5 |
| 4 Stunden | 175 | 25 |

Die vorliegende Tabelle beweist sehr augenfällig, daß bereits nach 30 Minuten nach Auftragen der Zusammensetzung 4 alle 20 Probanden über eine hohe Schmerzunempfindlichkeit gegenüber den zuvor beschriebenen Einstichen berichteten, wobei diese Schmerzunempfindlichkeit über zwei Stunden nahezu unverändert erhalten blieb. Erst nach vier Stunden konnte ein Abfall der Schmerzunempfindlichkeit festgestellt werden.

### Ausführungsbeispiel 5

### insulinhaltige gelartige Zusammensetzung

Es wird eine pharmazeutische Zusammensetzung aus den nachfolgend aufgelisteten Inhaltsstoffen erstellt:

**Phase 1**

| | |
|---|---|
| hydriertes Phosphatidylcholin, Konzentration anhydriertem Phosphatidylcholin 90 Gew.% | 1,95 % |
| C12-C16-Alkylbenzoate | 16,0 % |
| Palmitinsäure | 0,8 % |
| Cetearylalkohol | 0,3 % |
| Insulin (Hoechst, 40IU/ml) | 1,5 % |
| Ethanol | 10,0 % |

**Phase 2**

| | |
|---|---|
| Propylenglykol | 6,0 % |
| Glycerin | 5,0 % |
| Acetamid MEA | 0,5 % |
| Betain, wasserfrei | 0,3 % |
| Sarcosin | 0,7 % |
| Wasser DAB 10 | ad 100,0 % |

Zur Herstellung wurden zunächst die Phase 1 und die Phase 2 auf 75 °C erwärmt. Hiernach wurde die Phase 2 zu der Phase 1 unter Beibehaltung der Temperatur langsam zugesetzt, während die Mischung kontinuierlich gerührt wurde. Nachdem eine vollständige Mischung erstellt war, wurde diese Mischung während zwei Minuten unter Verwendung eines Homogenisators (Ultra Turrax) während 20.000 U/min homogenisiert.

An diese Homogenisierung schloß sich eine acht Minuten dauernde Zwangshomogenisierung mittels Hochdruckhomogenisation bei 790 bar an. Anschließend wurde die Mischung unter kontinuierlichem Rühren auf 37 °C gekühlt. Hiernach erfolgte eine erneute Homogenisation während drei Minuten unter Verwendung eines Ultra Turrax-Homogenisators bei 14.000 U/min. Unter kontinuierlichem Rühren wurde danach die Mischung auf Raumtemperatur abgekühlt.

Zum Nachweis der Wirksamkeit der Zusammensetzung 5 wurde ein männlicher Proband mit einer Gewicht von 80 kg und einem Alter von 45 Jahren mit einem Gramm der Zusammensetzung 5 behandelt, wobei diese Menge der Zusammensetzung 5 auf eine Fläche von 15 cm² Haut am Unterarm aufgetragen und leicht einmassiert wurde.

Vor der Applikation der Zusammensetzung 5 und nach einer Verweilzeit von 30 Minuten nach der Applikation wurden jeweils der Blutzucker bestimmt. Der Abfall des Blutzuckers betrug 5 %, bezogen auf den ursprünglichen Zuckerwert.

Nach einer weiteren Verweilzeit von 120 Minuten wurde ein Blutzuckerabfall von 30 %, bezogen auf den ursprünglichen Wert, bestimmt. Nach weiteren 120 Minuten betrug der Blutzuckerabfall 40 %, bezogen auf den ursprünglichen Wert, wobei erst nach weiteren fünf Stunden, in denen der Blutzuckerwert innerhalb der Toleranz 40 % unter dem ursprünglichen Zuckerwert lag, der Blutzuckerwert innerhalb von zwei weiteren Stunden auf den Ausgangsblutzuckerwert anstieg.

Alle zuvor in den Ausführungsbeispielen 1 bis 5 angegebenen %-Werte beziehen sich auf Gewichtsprozent, während Sarcosin chemisch korrekt als Methylglycin zu bezeichnen ist.

## Patentansprüche

1. Pharmazeutische Zusammensetzung, enthaltend
a) Wasser,
b) mindestens einen topisch applizierbaren Wirkstoff,
c) mindestens eine Trägersubstanz für den Wirkstoff, wobei die Trägersubstanz eine mit Wasser eine lamellare Doppelmembranstruktur ausbildende Substanz ist,
d) mindestens eine Verbindung, die wenigstens eine funktionelle Gruppe der allgemeinen Formel enthält
- CH₂ - N^{⊕} - (CH₃)₃ (Formel I),
wobei diese Verbindung aus der Gruppe ausgewählt ist, die Betain, Acetylcholin, Cholin, Glycerophosphocholin, Phosphatidylcholin, Lysophosphatidylcholin, Carnitin, Acylcarnitin und Sphingomyeline umfasst, und/oder S-Adenosylmethionin, und
e) mindestens ein N-Acyl-Ethanolamin,
f) wobei diese Zusammensetzung des weiteren eine solche lamellare Doppelmembranstruktur aufweist, die einen derartigen Schichtaufbau besitzt, daß eine obere Schicht der Trägersubstanz so zu einer unteren Schicht der Trägersubstanz ausgerichtet ist, dass die hydrophilen Reste der Trägersubstanz jeweils nach außen zu der die Trägersubstanzschichten umgebenden wäßrigen Phasen weisen, während die lipophilen Reste der Trägersubstanz zueinander nach innen ausgerichtet sind, wobei die Zusammensetzung geschichtete Doppelmembranen umfasst, welche mindestens zwei überwiegend planar verlaufende und jeweils durch mindestens eine Wasserschicht getrennte Doppelmembranen enthalten, und die Trägersubstanz ein hydriertes Phospholipid enthält, welches wenigstens 60 Gew.% hydriertes Phosphatidylcholin aufweist.

2. Pharmazeutische Zusammensetzung nach Anspruch 1, **dadurch gekennzeichnet, daß** die Zusammensetzung die Trägersubstanz in einer Konzentration zwischen 0,5 Gew.% und 30 Gew.%, vorzugsweise in einer Konzentration zwischen 1 Gew.% und 15 Gew.%, bezogen auf die anwendungsfertige Zusammensetzung, aufweist.

3. Pharmazeutische Zusammensetzung nach Anspruch 1 oder 2, **dadurch gekennzeichnet, daß** das hydrierte Phospholipid eine Phasenübergangstemperatur über 30 °C und unter 70 °C besitzt.

4. Pharmazeutische Zusammensetzung nach einem der vorangehenden Ansprüche, **dadurch gekennzeichnet, daß** die Zusammensetzung des weiteren Methylglycin, Dimethylglycin und/oder Methylmethionin enthält.

5. Pharmazeutische Zusammensetzung nach Anspruch 4, **dadurch gekennzeichnet, daß** das Methylglycin, Dimethylglycin und/oder Methylmethionin in der Zusammensetzung in einer Konzentration zwischen 0,0001 % und 10 %, vorzugsweise zwischen 0,1 % und 9 %, bezogen auf das Gewicht der anwendungsfertigen Zusammensetzung, vorhanden ist.

6. Pharmazeutische Zusammensetzung nach einem der vorangehenden Ansprüche, **dadurch gekennzeichnet, daß** die Verbindung und/oder das S-Adenosylmethionin in der Zusammensetzung in einer Konzentration zwischen 0,0001 % und 10 %, vorzugsweise zwischen 0,1 % und 9 %, bezogen auf das Gewicht der anwendungsfertigen Zusammensetzung, vorhanden ist.

7. Pharmazeutische Zusammensetzung nach einem der vorangehenden Ansprüche, **dadurch gekennzeichnet, daß** die Zusammensetzung eine Mischung enthält, die als Inhaltsstoffe Betain, Methylglycin und/oder N-Acyl-Ethanolamin umfaßt.

8. Pharmazeutische Zusammensetzung nach Anspruch 7, **dadurch gekennzeichnet, daß** die Mol-Massen in der zwei Inhaltsstoffe aufweisenden Mischung von 1:1 bis 1:9 variieren.

9. Pharmazeutische Zusammensetzung nach einem der vorangehenden Ansprüche, **dadurch gekennzeichnet, daß** die Zusammensetzung als Wirkstoff einen solchen Wirkstoff enthält, der aus der Gruppe ausgewählt ist, die Analgetika, Antirheumatika, Antiallergika, Antibiotika, Antimykotika, Antiphlogistika, Balneotherapeutika, corticoide Wirkstoffe, Dermatika, Antiseptika, durchblutungsfördernde Wirkstoffe, Sedativa, Anästhetika, Spasmolytika, Vitamine und Wundbehandlungsmittel jeweils allein oder in Mischung umfaßt.

10. Pharmazeutische Zusammensetzung nach Anspruch 9, **dadurch gekennzeichnet, daß** der mindestens eine Wirkstoff ein lokal wirkendes Dermatikum ist.

11. Pharmazeutische Zusammensetzung nach einem der vorangehenden Ansprüche, **dadurch gekennzeichnet, daß** der Wirkstoff in der Zusammensetzung in einer Konzentration zwischen 0,05 % und 20 %, vorzugsweise in einer Konzentration zwischen 0,1 % und 4 %, bezogen auf das Gewicht der anwendungsfertigen Zusammensetzung, vorhanden ist.

12. Pharmazeutische Zusammensetzung nach einem der vorangehenden Ansprüche, **dadurch gekennzeichnet, daß** die Zusammensetzung als Verbindung eine Fettsäure und/oder ein Fettsäuresalz des Betains und/oder eine Mischung aus Betain mit mindestens einer Fettsäure und/oder einem Fettsäuresalz enthält.

13. Pharmazeutische Zusammensetzung nach einem der vorangehenden Ansprüche, **dadurch gekennzeichnet, daß** die Zusammensetzung als Verbindung mindestens ein Fettsäuresalz des Betains aufweist.

14. Pharmazeutische Zusammensetzung nach Anspruch 12 oder 13, **dadurch gekennzeichnet, daß** die Fettsäure bzw. das Fettsäuresalz eine Kohlenstoffhauptkette mit 12 bis 22 Kohlenstoffatomen besitzt.

15. Pharmazeutische Zusammensetzung nach Anspruch 13 oder 14, **dadurch gekennzeichnet, daß** in der Zusammensetzung als Fettsäuresalz des Betains ein Betainlaurat, ein Betainmyristat, ein Betainpalmitat, ein Betainstearat, ein Betainoleat und/oder ein Betainlinolat vorhanden ist.

16. Pharmazeutische Zusammensetzung nach einem der vorangehenden Ansprüche, **dadurch gekennzeichnet, daß** die Zusammensetzung Wasser in einer Konzentration zwischen 5 % und 90 %, bezogen auf das Gewicht der anwendungsfertigen Zusammensetzung, aufweist.

17. Pharmazeutische Zusammensetzung nach einem der vorangehenden Ansprüche, **dadurch gekennzeichnet, daß** die Zusammensetzung des weiteren mindestens ein Konservierungsmittel, ein Antioxidans, ein Verdickungsmittel, einen Gelbildner, eine osmoaktive Substanz, einen sonstigen Zusatzstoff und/oder einen Alkohol, vorzugsweise einen mehrwertigen Alkohol, aufweist.

18. Pharmazeutische Zusammensetzung nach einem der vorangehenden Ansprüche, **dadurch gekennzeichnet, daß** die Zusammensetzung das N-Acyl-Ethanolamin in einer Konzentration zwischen 0,01 % und 10 %, vorzugsweise zwischen 0,1 % und 3 %, bezogen auf das Gewicht der anwendungsfertigen Zusammensetzung, aufweist.

19. Pharmazeutische Zusammensetzung nach einem der vorangehenden Ansprüche, **dadurch gekennzeichnet, daß** der Acylrest des N-Acyl-Ethanolamins ein C₁-C₁₈-Acylrest ist.

20. Pharmazeutische Zusammensetzung nach einem der vorangehenden Ansprüche, **dadurch gekennzeichnet, daß** diese als N-Acyl-Ethanolamin, N-Acetyl-Ethanolamin, N-Oleoyl-Ethanolamin, N-Linolenoyl-Ethanolamin, N-Cocoyl-Ethanolamin und/oder N-Palmitinoyl-Ethanolamin enthält.

21. Pharmazeutische Zusammensetzung nach einem der vorangehenden Ansprüche, **dadurch gekennzeichnet, daß** die Zusammensetzung zwischen
5 % und 90 % Wasser,
1 % und 15 % Trägersubstanz,
0,0001 % bis 10 % der Verbindung und/oder des S-Adenosylmethionins,
0,05 % bis 20 % des mindestens einen Wirkstoffes,
0,01 % und 10 % des N-Acyl-Ethanolamins
sowie übliche sonstige Bestandteile in einer Konzentration zwischen 0 % und 60 % enthält, wobei sich die zuvor wiedergegebenen Konzentrationsangaben auf das Gewicht der anwendungsfertigen Zusammensetzung beziehen.

22. Pharmazeutische Zusammensetzung nach einem der vorangehenden Ansprüche, **dadurch gekennzeichnet, daß** die Zusammensetzung als topisch applizierbares Gel formuliert ist und eine Viskosität bei 20 °C zwischen 4.000 mPas•s und 40.000 mPas•s, vorzugsweise zwischen 12.000 mPas•s und 25.000 mPas•s, aufweist.

23. Pharmazeutische Zusammensetzung nach einem der vorangehenden Ansprüche, **dadurch gekennzeichnet, daß** die Zusammensetzung einen pH-Wert zwischen 4,0 und 7,6 besitzt.

24. Pharmazeutische Zusammensetzung nach einem der vorangehenden Ansprüche, **dadurch gekennzeichnet, daß** die Zusammensetzung die lamellare Doppelmembranstruktur in einer Konzentration zwischen 15 % und 95 %, vorzugsweise zwischen 30 % und 95 %, bezogen auf das Gewicht der in der Zusammensetzung enthaltenen Trägersubstanz, aufweist.

25. Pharmazeutische Zusammensetzung nach einem der vorangehenden Ansprüche, **dadurch gekennzeichnet, daß** die Zusammensetzung eine solche lamellare Doppelmembranstruktur aufweist, bei der jede einzelne Doppelmembran eine Dicke zwischen 4 nm und 20 nm, vorzugsweise zwischen 4 nm und 8 nm, besitzt.

26. Pharmazeutische Zusammensetzung nach einem der vorangehenden Ansprüche, **dadurch gekennzeichnet, daß** die Zusammensetzung als Wirkstoff wenigstens ein Vitamin, vorzugsweise ein Vitamin E, allein oder in Mischung mit anderen Vitaminen, enthält.

27. Transdermal zu verabreichende Zubereitung, **dadurch gekennzeichnet, daß** die Zubereitung als Wirkstoffreservoir eine pharmazeutische Zusammensetzung nach einem der vorangehenden Ansprüche 1 bis 26 aufweist.

28. Transdermal zu verabreichende Zubereitung nach Anspruch 27, **dadurch gekennzeichnet, daß** die Zubereitung eine solche pharmazeutische Zusammensetzung enthält, die neben mindestens einer Trägersubstanz und Wasser des weiteren
a) Cholin, Betain, Carnitin, Methylmethionin und/oder Phosphatidylcholin,
b) N-Acyl-Ethanolamin mit einem C₁₀-C₁₈-Acylrest,
c) Methylglycin und
d) mindestens einen transdermal zu verabreichenden, in die Haut penetrierenden und/oder die Haut permeatierenden Wirkstoff aufweist.

29. Transdermal zu verabreichende Zubereitung nach Anspruch 28, **dadurch gekennzeichnet, daß** das Massenverhältnis der Komponente a) zur Komponente b) und zur Komponente c) zwischen 1:1:1 und 1:10:1 variiert.

30. Transdermal zu verabreichende Zubereitung nach Anspruch 28, **dadurch gekennzeichnet, daß** das Massenverhältnis der Komponente a) zur Komponente b) und zur Komponente c) zwischen 1:1:1 und 10:1:1 variiert.

31. Transdermal zu verabreichende Zubereitung nach Anspruch 28, **dadurch gekennzeichnet, daß** das Massenverhältnis der Komponente a) zur Komponente b) und zur Komponente c) zwischen 1:1:1 und 1:1:10 variiert.

32. Transdermal zu verabreichende Zubereitung nach einem der Ansprüche 27 bis 31, **dadurch gekennzeichnet, daß** der Wirkstoff ein systemisch wirkender Wirkstoff ist.

33. Transdermal zu verabreichende Zubereitung nach einem der Ansprüche 27 bis 32, **dadurch gekennzeichnet, daß** die Zubereitung neben dem Wirkstoffreservoir des weiteren noch ein das Wirkstoffreservoir abdeckendes Schutzelement umfaßt.

## Claims

1. Pharmaceutical composition, containing
a) water,
b) at least one topically applicable active agent,
c) at least one carrier substance for the active agent, wherein the carrier substance is a substance forming a lamellar double membrane structure with water,
d) at least one compound containing at least one functional group of the general formula I
- CH₂ - N^{⊕} - (CH₃)₃ (formula I),
wherein this compound is selected from the group comprising betaine, acetylcholine, choline, glycerophosphocholine, phosphatidylcholine, lysophosphatidylcholine, carnitine, acylcarnitine and sphingomyeline, and/or S-adenosylmethionine, and
e) at least one N-acyl-ethanolamine,
f) wherein this composition further contains a lamellar double membrane structure such that it has a layer structure in which an upper layer of the carrier substance is oriented towards a lower layer of the carrier substance such that the hydrophilic residues of the carrier substance each point outwards to the aqueous phases surrounding the carrier substance layers, while the lipophilic residues of the carrier substance are oriented inwards towards each other, wherein the composition comprises layered double membranes, containing at least two double membranes which are mostly planar and each separated by at least one water layer, and the carrier substance contains a hydrogenated phospholipid having at least 60% by weight of hydrogenated phosphatidylcholine.

2. Pharmaceutical composition according to claim 1, **characterized in that** the composition contains the carrier substance in a concentration of between 0.5% by weight and 30% by weight, preferably in a concentration of between 1% by weight and 15% by weight, based on the ready-to-use composition.

3. Pharmaceutical composition according to claim 1 or 2, **characterized in that** the hydrogenated phospholipid has a phase transition temperature of above 30°C and below 70°C.

4. Pharmaceutical composition according to any of the preceding claims, **characterized in that** the composition further contains methylglycine, dimethylglycine and/or methylmethionine.

5. Pharmaceutical composition according to claim 4, **characterized in that** the methylglycine, dimethylglycine and/or methylmethionine is present in the composition in a concentration of between 0.0001% and 10%, preferably between 0.1% and 9%, based on the weight of the ready-to-use composition.

6. Pharmaceutical composition according to any of the preceding claims, **characterized in that** the compound and/or the S-adenosylmethionine is present in the composition in a concentration of between 0.0001% and 10%, preferably between 0.1% and 9%, based on the weight of the ready-to-use composition.

7. Pharmaceutical composition according to any of the preceding claims, **characterized in that** the composition contains a mixture comprising as ingredients betaine, methylglycine and/or N-acyl-ethanolamine.

8. Pharmaceutical composition according to claim 7, **characterized in that** the molar masses in the mixture containing two ingredients vary from 1:1 to 1:9.

9. Pharmaceutical composition according to any of the preceding claims, **characterized in that** the composition contains as an active agent such an active agent selected from the group comprising analgetics, antirheumatic drugs, antiallergics, antibiotics, antimycotics, antiphlogistics, balneotherapeutics, corticoid agents, dermatological agents, antiseptics, blood circulation-promoting active agents, sedativs, anesthetics, spasmolytics, vitamins and wound treating agents alone or in a mixture.

10. Pharmaceutical composition according to claim 9, **characterized in that** the at least one active agent is a locally active dermatological agent.

11. Pharmaceutical composition according to any of the preceding claims, **characterized in that** the active agent is present in the composition in a concentration of between 0.05% and 20%, preferably in a concentration of between 0.1% and 4%, based on the weight of the ready-to-use composition.

12. Pharmaceutical composition according to any of the preceding claims, characterized that the composition contains as a compound a fatty acid and/or a fatty acid salt of the betaine and/or a mixture of betaine with at least one fatty acid and/or fatty acid salt.

13. Pharmaceutical composition according to any of the preceding claims, **characterized in that** the composition contains as a compound at least one fatty acid salt of the betaine.

14. Pharmaceutical composition according to claim 12 or 13, **characterized in that** the fatty acid or the fatty acid salt contains a carbon main chain having 12 to 22 carbon atoms.

15. Pharmaceutical composition according to claim 13 or 14, **characterized in that** the composition contains as a fatty acid salt of the betaine a betaine laureate, a betaine myristate, a betaine palmitate, a betaine stearate, a betaine oleate and/or a betaine linolate.

16. Pharmaceutical composition according to any of the preceding claims, **characterized in that** the composition contains water in a concentration of between 5% and 90%, based on the weight of the ready-to-use composition.

17. Pharmaceutical composition according to any of the preceding claims, **characterized in that** the composition further contains at least one preservative, an antioxidant, a thickener, a gelling agent, an osmoactive substance, another additive and/or an alcohol, preferably a polyvalent alcohol.

18. Pharmaceutical composition according to any of the preceding claims, **characterized in that** the composition contains the N-acyl-ethanolamine in a concentration of between 0.01% and 10%, preferably between 0.1% and 3%, based on the weight of the ready-to-use composition.

19. Pharmaceutical composition according to any of the preceding claims, **characterized in that** the acyl group of the N-acyl-ethanonlamine is a C₁-C₁₈ acyl group.

20. Pharmaceutical composition according to any of the preceding claims, **characterized in that** it contains as N-acyl-ethanolamine, N-acetyl-ethanolamine, N-oleoyl-ethanolamine, N-linolenoyl-ethanolamine, N-cocoyl-ethanolamine and/or N-palmitinoyl-ethanolamine.

21. Pharmaceutical composition according to any of the preceding claims, **characterized in that** the composition contains between
5 % and 90 % water,
1 % and 15 % carrier substance,
0.0001 % to 10 % of the compound and/or of the S-adenosyl methionine,
0.05 % to 20 % of the at least one active agent,
0.01 % and 10 % of the N-acyl-ethanolamine
as well as other common ingredients in a concentration of between 0% and 60% the indications with respect to the concentration made above being based on the weight of the ready-to-use composition.

22. Pharmaceutical composition according to any of the preceding claims, **characterized in that** the composition is formulated as a gel for topical application and that it has a viscosity at 20°C of between 4,000 mPas•s and 40,000 mPas•s, preferably between 12,000 mPas•s and 25,000 mPas•s.

23. Pharmaceutical composition according to any of the preceding claims, **characterized in that** the composition has a pH value of between 4.0 and 7.6.

24. Pharmaceutical composition according to any of the preceding claims, **characterized in that** the composition contains the lamellar double membrane structure in a concentration of between 15% and 95%, preferably between 30% and 95%, based on the weight of the carrier substance contained in the composition.

25. Pharmaceutical composition according to any of the preceding claims, **characterized in that** the composition has such a lamellar double membrane structure in which every single double membrane has a thickness of between 4 nm and 20 nm, preferably between 4 nm and 8 nm.

26. Pharmaceutical composition according to any of the preceding claims, **characterized in that** the composition contains as an active agent at least one vitamin, preferably one vitamin E, alone ore in mixture with other vitamins.

27. Transdermally administerable preparation, **characterized in that** the preparation contains as an active agent reservoir a pharmaceutical composition according to any of preceding claims 1 to 26.

28. Transdermally administerable composition according to claim 27, **characterized in that** the preparation contains such a pharmaceutical composition which, apart from at least one carrier substance and water, further contains
a) choline, betaine, carnitine, methylmethionine and/or phosphatidylcholine,
b) N-acyl-ethanolamine having a C₁₀-C₁₈ acyl group,
c) methylglycine and
d) at least one transdermally administerable skin-penetrating and/or skin-permeating acitive agent.

29. Transdermally administerable preparation according to claim 28, **characterized in that** the mass ratio of component a) to component b) and to component c) varies between 1:1:1 and 1:10:1.

30. Transdermally administerable preparation according to claim 28, **characterized in that** the mass ratio of component a) to component b) and to component c) varies between 1:1:1 and 10:1:1.

31. Transdermally administerable preparation according to claim 28, **characterized in that** the mass ratio of component a) to component b) and to component c) varies between 1:1:1 and 1:1:10.

32. Transdermally administerable preparation according to any of claims 27 to 31, **characterized in that** the active agent is a systemically acting active agent.

33. Transdermally administerable preparation according to any of claims 27 to 32, **characterized in that** the preparation, apart from the active agent reservoir, further contains a protective element covering the active agent reservoir.

## Revendications

1. Composition pharmaceutique contenant
a) de l'eau,
b) au moins un principe actif applicable par voie topique,
c) au moins une substance de support pour le principe actif, laquelle substance de support est une substance formant une structure de membrane double lamellaire avec l'eau,
d) au moins un composé contenant au moins un groupe fonctionnel de formule générale I
- (CH₂) -N^{⊕} - (CH₃)₃ (formule I)
lequel composé est choisi dans l'ensemble comprenant la bétaïne, l'acétylcholine, la choline, la glycérophosphocholine, la phosphatidylcholine, la lysophosphatidylcholine, la carnitine, l'acylcarnitine et la spingomyéline, et/ou la S-adénosylméthionine, et
e) au moins une N-acyl-éthanolamine,
f) laquelle composition contient en outre une structure de membrane double lamellaire telle qu'elle ait une structure stratifiée dans laquelle une couche supérieure de la substance de support est orientée en direction d'une couche inférieure de la substance de support de façon que chacun des résidus hydrophiles de la substance de support pointe vers l'extérieur en direction des phases aqueuses entourant les couches de substance de support, tandis que les résidus lipophiles de la substance de support sont orientés vers l'intérieur en direction les uns des autres, et laquelle composition comprend des membranes doubles stratifiées, contenant au moins deux membranes doubles qui sont principalement planes et sont séparées chacune par au moins une couche d'eau, et la substance de support contient un phospholipide hydrogéné ayant au moins 60 % en poids de phosphatidylcholine hydrogénée.

2. Composition pharmaceutique selon la revendication 1, **caractérisée en ce qu'**elle contient la substance de support à une concentration comprise entre 0,5 % en poids et 30 % en poids, de préférence à une concentration comprise entre 1 % en poids et 15 % en poids, par rapport à la composition prête à l'emploi.

3. Composition pharmaceutique selon la revendication 1 ou 2, **caractérisée en ce que** le phospholipide hydrogéné a une température de transition vitreuse supérieure à 30°C et inférieure à 70°C.

4. Composition pharmaceutique selon l'une quelconque des revendications précédentes, **caractérisée en ce qu'**elle contient en outre de la méthylglycine, de la diméthylglycine et/ou de la méthylméthionine.

5. Composition pharmaceutique selon la revendication 4, **caractérisée en ce que** la méthylglycine, la diméthylglycine et/ou la méthylméthionine sont présentes dans la composition à une concentration comprise entre 0,0001 % et 10 %, de préférence entre 0,1 % et 9 %, par rapport au poids de la composition prête à l'emploi.

6. Composition pharmaceutique selon l'une quelconque des revendications précédentes, **caractérisée en ce que** le composé et/ou la S-adénosylméthionine sont présents dans la composition à une concentration comprise entre 0,0001 % et 10 %, de préférence entre 0,1 % et 9 %, par rapport au poids de la composition prête à l'emploi.

7. Composition pharmaceutique selon l'une quelconque des revendications précédentes, **caractérisée en ce qu'**elle contient un mélange comprenant, en tant qu'ingrédients, de la bétaïne, de la méthylglycine et/ou de la N-acyl-éthanolamine.

8. Composition pharmaceutique selon la revendication 7, **caractérisée en ce que** les masses molaires dans le mélange contenant deux ingrédients varient de 1/1 à 1/9.

9. Composition pharmaceutique selon l'une quelconque des revendications précédentes, **caractérisée en ce qu'**elle contient, en tant que principe actif, un principe actif choisi dans le groupe comprenant les analgésiques, les antirhumatismaux, les antiallergiques, les antibiotiques, les antimycotiques, les antiphlogistiques, les balnéothérapeutiques, les corticoïdes, les agents dermatologiques, les antiseptiques, les principes actifs favorisant la circulation sanguine, les sédatifs, les anesthésiants, les spasmolytiques, les vitamines et les agents de traitement des plaies, seul ou en mélange.

10. Composition pharmaceutique selon la revendication 9, **caractérisée en ce que** l'au moins un principe actif est un agent dermatologique à action locale.

11. Composition pharmaceutique selon l'une quelconque des revendications précédentes, **caractérisée en ce que** le principe actif est présent dans la composition à une concentration comprise entre 0,05 % et 20 %, de préférence à une concentration comprise entre 0,1 % et 4 %, par rapport au poids de la composition prête à l'emploi.

12. Composition pharmaceutique selon l'une quelconque des revendications précédentes, **caractérisée en ce qu'**elle contient, en tant que composé, un acide gras et/ou un sel d'acide gras de bétaïne et/ou un mélange de bétaïne avec au moins un acide gras et/ou un sel d'acide gras.

13. Composition pharmaceutique selon l'une quelconque des revendications précédentes, **caractérisée en ce qu'**elle contient, en tant que composé, au moins un sel d'acide gras de bétaïne.

14. Composition pharmaceutique selon la revendication 12 ou 13, **caractérisée en ce que** l'acide gras ou le sel d'acide gras contient une chaîne principale carbonée ayant 12 à 22 atomes de carbone.

15. Composition pharmaceutique selon la revendication 13 ou 14, **caractérisée en ce qu'**elle contient, en tant que sel d'acide gras de bétaïne, du laurate de bétaïne, du myristate de bétaïne, du palmitate de bétaïne, du stéarate de bétaïne, de l'oléate de bétaïne et/ou du linoléate de bétaïne.

16. Composition pharmaceutique selon l'une quelconque des revendications précédentes, **caractérisée en ce qu'**elle contient de l'eau à une concentration comprise entre 5 % et 90 % par rapport au poids de la composition prête à l'emploi.

17. Composition pharmaceutique selon l'une quelconque des revendications précédentes, **caractérisée en ce qu'**elle contient en outre au moins un conservateur, un antioxydant, un épaississant, un agent gélifiant, une substance osmo-active, un autre additif et/ou un alcool, de préférence un alcool polyvalent.

18. Composition pharmaceutique selon l'une quelconque des revendications précédentes, **caractérisée en ce qu'**elle contient la N-acyl-éthanolamine à une concentration comprise entre 0,01 % et 10 %, de préférence entre 0,1 % et 3 %, par rapport au poids de la composition prête à l'emploi.

19. Composition pharmaceutique selon l'une quelconque des revendications précédentes, **caractérisée en ce que** le groupe acyle de la N-acyl-éthanolamine est un groupe acyle en C₁ à C₁₈.

20. Composition pharmaceutique selon l'une quelconque des revendications précédentes, **caractérisée en ce qu'**elle contient, en tant que N-acyl-éthanolamine, de la N-acétyl-éthanolamine, de la N-oléoyl-éthanolamine, de la N-linolénoyl-éthanolamine, de la N-cocoyl-éthanolamine et/ou de la N-palmitinoyl-éthanolamine.

21. Composition pharmaceutique selon l'une quelconque des revendications précédentes, **caractérisée en ce qu'**elle contient entre
5 % et 90 % d'eau,
1 % et 15 % de substance de support,
0,0001 % et 10 % du composé et/ou de la S-adénosyl-méthionine,
0,05 % et 20 % de l'au moins un principe actif,
0,01 % et 10 % de la N-acyl-éthanolamine,
ainsi que d'autres ingrédients courants à une concentration comprise entre 0 % et 60 %,
les indications concernant la concentration apportées ci-dessus étant basées sur le poids de la composition prête à l'emploi.

22. Composition pharmaceutique selon l'une quelconque des revendications précédentes, **caractérisée en ce qu'**elle est formulée en un gel pour application par voie topique et **en ce qu'**elle a une viscosité à 20°C comprise entre 4 000 mPa·s et 40 000 mPa·s, de préférence entre 12 000 mPa·s et 25 000 mPa·s.

23. Composition pharmaceutique selon l'une quelconque des revendications précédentes, **caractérisée en ce qu'**elle a une valeur de pH comprise entre 4,0 et 7,6.

24. Composition pharmaceutique selon l'une quelconque des revendications précédentes, **caractérisée en ce qu'**elle contient la structure de membrane double lamellaire à une concentration comprise entre 15 % et 95 %, de préférence entre 30 % et 95 %, par rapport au poids de la substance de support contenue dans la composition.

25. Composition pharmaceutique selon l'une quelconque des revendications précédentes, **caractérisée en ce qu'**elle a une telle structure de membrane double lamellaire dans laquelle chaque membrane double isolée a une épaisseur comprise entre 4 nm et 20 nm, de préférence entre 4 nm et 8 nm.

26. Composition pharmaceutique selon l'une quelconque des revendications précédentes, **caractérisée en ce qu'**elle contient, en tant que principe actif, au moins une vitamine, de préférence une vitamine E, seule ou en mélange avec d'autres vitamines.

27. Préparation pouvant être administrée par voie transdermique, **caractérisée en ce qu'**elle contient, en tant que réservoir de principe actif, une composition pharmaceutique selon l'une quelconque des revendications 1 à 26.

28. Préparation pouvant être administrée par voie transdermique selon la revendication 27, **caractérisée en ce qu'**elle contient une telle composition pharmaceutique qui, à part au moins une substance de support et de l'eau, contient en outre
a) de la choline, de la bétaïne, de la carnitine, de la méthylméthionine et/ou de la phosphatidylcholine,
b) une N-acyl-éthanolamine ayant un groupe acyle en C₁₀ à C₁₈,
c) de la méthylglycine et
d) au moins principe actif traversant la peau par perméation et/ou pénétrant dans la peau, pouvant être administré par voie transdermique.

29. Préparation pouvant être administrée par voie transdermique selon la revendication 28, **caractérisée en ce que** le rapport en masse du composant a) au composant b) et au composant c) varie entre 1/1/1 et 1/10/1.

30. Préparation pouvant être administrée par voie transdermique selon la revendication 28, **caractérisée en ce que** le rapport en masse du composant a) au composant b) et au composant c) varie entre 1/1/1 et 10/1/1.

31. Préparation pouvant être administrée par voie transdermique selon la revendication 28, **caractérisée en ce que** le rapport en masse du composant a) au composant b) et au composant c) varie entre 1/1/1 et 1/1/10.

32. Préparation pouvant être administrée par voie transdermique selon l'une quelconque des revendications 27 à 31, **caractérisée en ce que** le principe actif est un principe actif agissant par voie systémique.

33. Préparation pouvant être administrée par voie transdermique selon l'une quelconque des revendications 27 à 32, **caractérisée en ce que** la préparation, à part ce qui provient du réservoir de principe actif, contient en outre un élément protecteur couvrant le réservoir de principe actif.
